# EUROPEAN PATENT APPLICATION

(11) **EP 4 643 938 A1**
(43) Date of publication of application: **05.11.2025**
(21) Application number: 23912789.7
(22) Date of filing: 21.12.2023
(51) Int. Cl.: A61N 1/40, A61N 1/36, A61N 1/32, A61N 1/06, A61H 9/00, A61N 1/08

(54) **COSMETIC PROCEDURE DEVICE HAVING NON-CONTACT DETECTION SENSOR**

(30) Priority: 29.12.2022 KR 20220188300
(71) Applicant: APR CO., LTD., Seoul 05551 (KR)
(72) Inventor: JI, Jong Chul, Seoul 08592 (KR); LEE, Gyoun Jung, Seoul 08592 (KR); PARK, Seung Woo, Seoul 08592 (KR)
(74) Representative: Zermani Biondi Orsi, Umberto
(86) International application number: PCT/KR2023/021314
(87) International publication number: WO 2024/144114

(57) **Abstract**

A cosmetic procedure device is disclosed. A cosmetic procedure device, according to one aspect of the present invention, for performing a cosmetic procedure on the skin of a user may comprise: a main body; a procedure unit which is provided in the main body, and which has, therein, a procedure space in which the skin of the user undergoes a procedure; a stimulation application unit which is disposed in the procedure space, and which applies stimulation to the skin of the user; and a sensor unit which is disposed in the procedure unit, and which detects changes in capacitance so as to sense when the skin of the user is in contact with the stimulation application unit.

## Description

### TECHNICAL FIELD

The present invention relates to a cosmetic procedure device, and more particularly, to a cosmetic procedure device having a non-contact detection sensor that detects the height of raised skin without contacting the skin of a user.

### BACKGROUND

In recent years, home-use cosmetic procedure devices that allow users to perform cosmetic procedures such as massage and hair removal by themselves at home have become increasingly widespread. Home-use cosmetic procedure devices enable users to conveniently perform cosmetic procedures at their desired time in their own homes, thereby lowering the barrier to cosmetic procedures and improving accessibility.

With the increasing adoption of home-use cosmetic procedure devices, the performance of such devices has been significantly improved compared to conventional devices, and the effectiveness of the procedures has also been enhanced. This means that if the user operates the device improperly or fails to use it safely and appropriately, both the likelihood and severity of side effects may increase.

Specifically, a home-use cosmetic procedure device may perform procedures such as hair removal or massage using, for example, light or high-frequency energy. In particular, when high-frequency energy is applied to the skin for cosmetic or therapeutic purposes, it is essential that the high-frequency electrode be in precise contact with the skin to achieve the desired treatment effect and to generate deep heat.

However, if high-frequency energy is applied while the contact area between the main body of the cosmetic procedure device and the skin is reduced due to factors such as the contact angle, pressure variations, or curvature of the skin, the current density increases, posing a risk of burns.

Accordingly, as the performance and effectiveness of home-use cosmetic procedure devices have improved, there is a growing need to ensure that skin stimulation is enabled only when the contact surface of the main body of the cosmetic procedure device is in contact with the skin, in order to prevent risks caused by improper or unintended use of the device.

### DISCLOSURE

### TECHNICAL PROBLEM

The present invention is to solve the above problems, and the present invention is directed to providing a cosmetic procedure device that prevents excessive high-frequency stimulation from being applied to a user's skin by non-contact measurement of whether a stimulation application unit is in contact with the user's skin.

In addition, the present invention is also directed to providing a cosmetic procedure device that alleviates discomfort, skin stress, and the like caused by heat during cosmetic skin procedures.

The problems of the present invention are not limited to those mentioned above, and other problems not mentioned will be clearly understood by those of ordinary skill in the art from the following description.

### TECHNICAL SOLUTION

According to an aspect of the present disclosure, provided is a cosmetic procedure device for performing a cosmetic procedure on a user's skin, comprising a main body; a procedure unit provided in the main body, the procedure unit having a procedure space formed therein to perform a procedure on the user's skin; a stimulation application unit disposed in the procedure space, the stimulation application unit applying stimulation to the user's skin; and a sensor unit disposed in the procedure unit, the sensor unit sensing a state in which the user's skin is in contact with the stimulation application unit by detecting a change in capacitance.

In this case, the stimulation application unit may include a first electrode; and a second electrode disposed in parallel with and spaced apart from the first electrode.

In this case, the procedure unit may further include a partition that separates an interior of the main body from the procedure space, and the sensor unit may be disposed on a first side surface of the partition.

In this case, a support portion having a central groove communicating with the procedure space may be provided in a central region of the partition, and a temperature sensor configured to measure the temperature of the procedure space may be provided in the central groove.

In this case, the sensor unit may be formed to surround an outer surface of the support portion.

In this case, the sensor unit may include a first sensor unit having a ring shape; and a second sensor unit disposed to be spaced apart from the first sensor unit.

In this case, the cosmetic procedure device may further include a suction source disposed in the main body and configured to generate suction force; a suction flow path that forms negative pressure in the procedure space by drawing in air from the procedure space using the suction force of the suction source; and a control unit electrically connected to the sensor unit and configured to control the stimulation application unit and the suction source.

In this case, the suction flow path may include a plurality of suction ports arranged toward the procedure space, and the plurality of suction ports may include a first suction port formed on a second side surface of the partition, the second side surface being opposite to the first side surface of the partition; and a second suction port formed on the opposite side of the first suction port with the support portion therebetween.

### ADVANTAGEOUS EFFECT

According to the above configuration, the cosmetic procedure device according to an embodiment of the present invention senses the contact state between a user's skin and the stimulation application unit in a non-contact manner, thereby preventing excessive high-frequency stimulation from being applied to the user's skin by the stimulation application unit.

In addition, since the cosmetic procedure device is configured to apply high-frequency stimulation to the user's skin only when the user's skin is properly in contact with the stimulation application unit, the user can safely use the cosmetic procedure device.

### BRIEF DESCRIPTION OF THE DRAWINGS

FIG. 1 is a perspective view illustrating a cosmetic procedure device according to an embodiment of the present invention.
FIG. 2 is a perspective view illustrating the cosmetic procedure device according to an embodiment of the present invention from another angle.
FIG. 3 is a bottom view illustrating the cosmetic procedure device according to an embodiment of the present invention.
FIG. 4 is a cross-sectional view focusing on the procedure unit of the cosmetic procedure device according to an embodiment of the present invention.
FIG. 5 is an exploded perspective view illustrating the procedure unit, the stimulation application unit, and the sensor unit of the cosmetic procedure device according to an embodiment of the present invention.
FIG. 6 is a perspective view illustrating the procedure unit separated from the main body of the cosmetic procedure device according to an embodiment of the present invention.
FIG. 7 is a view illustrating a state in which the cosmetic procedure device according to an embodiment of the present invention performs a cosmetic procedure on the skin of a user.
FIG. 8 is a block diagram of the cosmetic procedure device according to an embodiment of the present invention.

100: cosmetic procedure device
110: main body
120: procedure unit
130: stimulation application unit
200: sensor unit

### MODES OF THE INVENTION

Hereinafter, exemplary embodiments of the present invention will be described in detail so that those of ordinary skill in the art can readily implement the present invention with reference to the accompanying drawings. The present invention may be embodied in many different forms and is not limited to the embodiments set forth herein. In the drawings, parts unrelated to the description are omitted for clarity of description of the present invention, and throughout the specification, same or similar reference numerals denote same elements.

Terms and words used in the present specification and claims should not be construed as limited to their usual or dictionary definition. They should be interpreted as meaning and concepts consistent with the technical idea of the present invention, based on the principle that inventors may appropriately define the terms and concepts to describe their own invention in the best way.

Accordingly, the embodiments described in the present specification and the configurations shown in the drawings correspond to preferred embodiments of the present invention, and do not represent all the technical idea of the present invention, so the configurations may have various examples of equivalent and modification that can replace them at the time of filing the present invention.

It should be understood that the terms "comprise or include" or "have" or the like when used in this specification, are intended to describe the presence of stated features, numbers, steps, operations, elements, components and/or a combination thereof but not preclude the possibility of the presence or addition of one or more other features, numbers, steps, operations, elements, components, or a combination thereof.

The presence of an element in/on "front", "rear", "upper or above or top" or "lower or below or bottom" of another element includes not only being disposed in/on "front", "rear", "upper or above or top" or "lower or below or bottom" directly in contact with other elements, but also cases in which another element being disposed in the middle, unless otherwise specified. In addition, unless otherwise specified, that an element is "connected" to another element includes not only direct connection to each other but also indirect connection to each other.

Hereinafter, a cosmetic procedure device according to an embodiment of the present invention will be described with reference to the drawings.

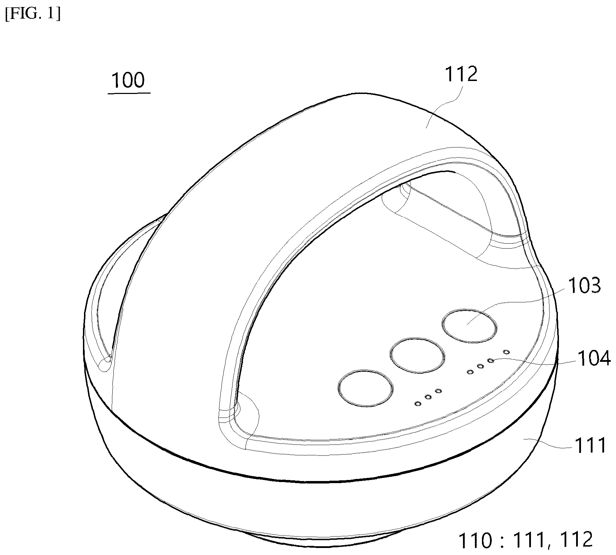

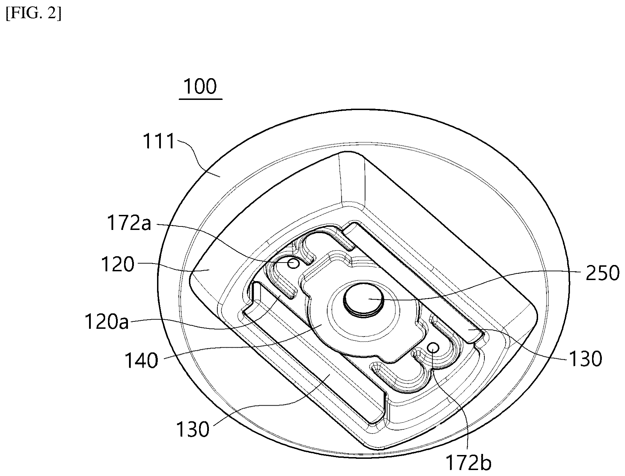

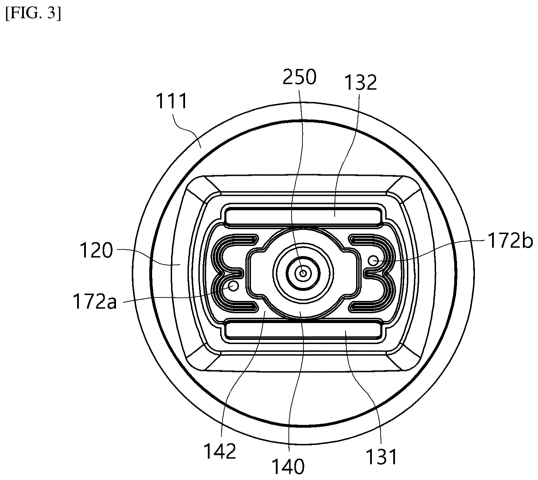

FIG. 1 is a perspective view illustrating a cosmetic procedure device according to an embodiment of the present invention, FIG. 2 is a perspective view illustrating the cosmetic procedure device according to an embodiment of the present invention from another angle, and FIG. 3 is a bottom view illustrating the cosmetic procedure device according to an embodiment of the present invention.

Referring to FIGs. 1 to 3, a cosmetic procedure device 100 according to an embodiment of the present invention is a cosmetic procedure device that allows a user to perform a cosmetic procedure on their own skin. Meanwhile, the user may also perform a cosmetic procedure on another person using the cosmetic procedure device 100.

For example, the cosmetic procedure device 100 according to an embodiment of the present invention may provide a suction function that stimulates a user's skin by applying high-frequency stimulation while suctioning the skin, may provide massage using high-frequency electrodes on a user's skin, or may perform a hair removal function.

Components for performing cosmetic procedures may be disposed in the main body 110. For example, the main body 110 may be provided with a stimulation application unit 130 for high-frequency stimulation or a light source for light irradiation. In addition, the main body 110 may also be provided with a component for implementing a suction function that stimulates the user's skin by suctioning it.

As shown in FIG. 1, a handle portion 112 may be provided on an upper side of the main body 110 so that the user can hold and move the cosmetic procedure device 100.

In addition, the main body 110 may be provided with an operation button 103 for receiving input of operation information such as turning the cosmetic procedure device 100 on or off and selecting various modes. This is merely an example, and instead of the operation button 103, a touch screen or the like may be provided on the main body 110.

Meanwhile, the cosmetic procedure device 100 according to an embodiment of the present invention may include an output unit 104 for outputting signals, messages, or other information to the user. In this case, the output unit 104 may be a speaker for outputting sound or voice.

In addition, a display for presenting visual information may be provided around the circumference 111 of the main body 110. Meanwhile, the display may also serve the function of indicating the movement direction of the main body 110.

Meanwhile, the cosmetic procedure device 100 according to an embodiment of the present invention includes a procedure unit 120 provided in the main body 110, in which a procedure space 120a is formed to perform a procedure on the skin of a user on the inner side.

The procedure unit 120 is disposed on the opposite side of the handle portion 112, with the circumference 111 of the main body 110 therebetween. In this case, the procedure unit 120 is formed such that the procedure space 120a has a rectangular parallelepiped shape. However, the procedure space 120a is not limited to having a rectangular parallelepiped shape, and may instead have various shapes such as a polyhedron, an elliptical cylinder, or a cylindrical shape.

Meanwhile, the procedure space 120a of the procedure unit 120 is provided with suction ports 172a and 172b that draw in air from the procedure space 120a in order to perform the suction function of the cosmetic procedure device 100. The detailed configuration for performing the suction function of the cosmetic procedure device 100 will be described later with reference to the drawings.

In addition, the cosmetic procedure device 100 according to an embodiment of the present invention includes a stimulation application unit 130 that applies stimulation to the skin of a user.

The stimulation application unit 130 is disposed in the procedure space 120a. In this case, the stimulation application unit 130 generates high-frequency stimulation by receiving power. In this case, as shown in FIG. 3, the stimulation application unit 130 includes a first electrode 131 and a second electrode 132.

The first electrode 131 is formed in the shape of a plate having a predetermined thickness.

The second electrode 132 is formed in the shape of a plate having a predetermined thickness. In addition, the second electrode 132 may have the same size and shape as the first electrode 131. In addition, the second electrode 132 is disposed to be spaced apart from the first electrode 131. In this case, the second electrode 132 is disposed in parallel with the first electrode 131. Accordingly, the skin of a user in a raised state is placed between the first electrode 131 and the second electrode 132, and the first electrode 131 and the second electrode 132 come into contact with the raised skin to apply high-frequency stimulation to the raised skin.

In addition, the cosmetic procedure device 100 according to an embodiment of the present invention is provided with a temperature sensor 250 that measures the temperature of the procedure space 120a. As shown in FIG. 3, the temperature sensor 250 is disposed in a central region of the procedure space 120a when viewed from below.

In this case, since the temperature sensor 250 is disposed in the central region of the procedure space 120a, which corresponds to the raised portion of a user's skin, the temperature sensor 250 has the advantage of accurately measuring the temperature adjacent to the raised skin.

In addition, the temperature sensor 250 measures the temperature of the procedure space 120a to prevent excessive heat from being transferred to a user's skin during the cosmetic procedure. The temperature sensor 250 will be described in more detail below with reference to the drawings.

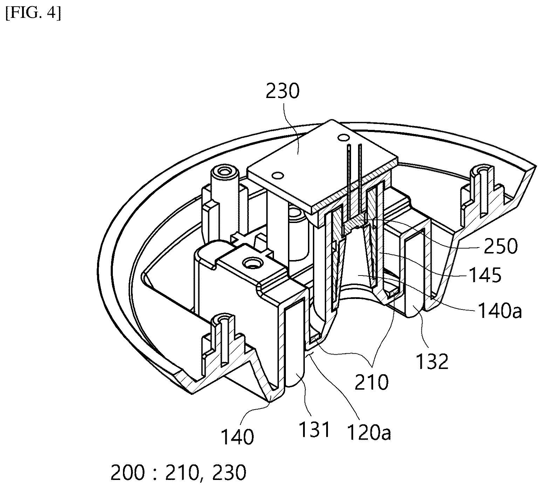

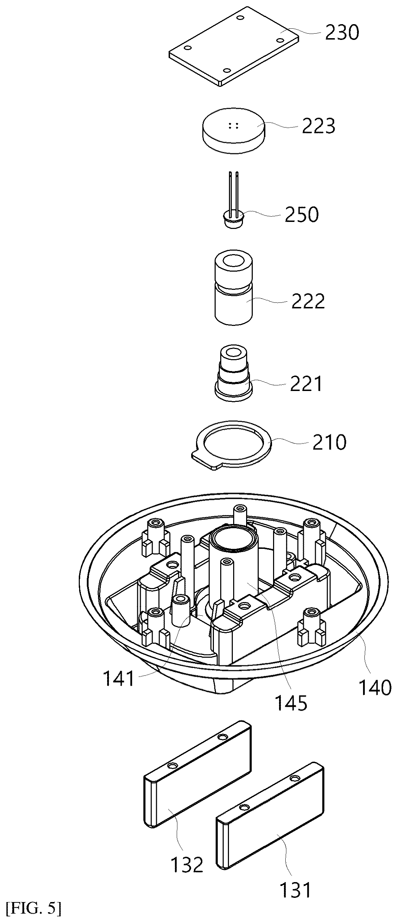

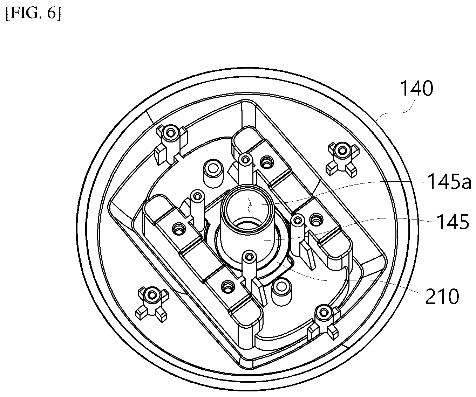

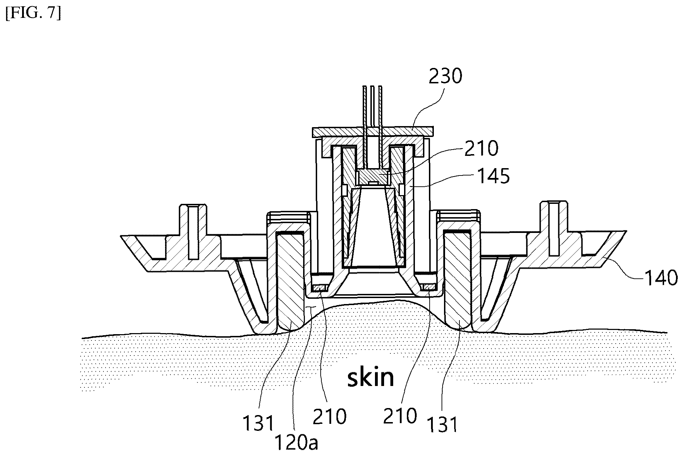

FIG. 4 is a cross-sectional view focusing on the procedure unit of the cosmetic procedure device according to an embodiment of the present invention, FIG. 5 is an exploded perspective view illustrating the procedure unit, the stimulation application unit, and the sensor unit of the cosmetic procedure device according to an embodiment of the present invention, FIG. 6 is a perspective view illustrating the procedure unit separated from the main body of the cosmetic procedure device according to an embodiment of the present invention, and FIG. 7 is a view illustrating a state in which the cosmetic procedure device according to an embodiment of the present invention performs a cosmetic procedure on the skin of a user.

Referring to FIGs. 4 to 7, the cosmetic procedure device 100 according to an embodiment of the present invention includes a sensor unit 200 that senses a state in which a user's skin is in contact with the first electrode 131 and the second electrode 132, which are included in the stimulation application unit 130.

The sensor unit 200 is disposed in the procedure unit 120 (see FIG. 2). In this case, the procedure unit 120 is provided with a partition 140 that separates the interior of the main body 100 (see FIG. 2) from the procedure space 120a.

In addition, the partition 140 has a first side surface 141 formed on the interior of the main body 110 and a second side surface 142 formed on the procedure space 120a. In addition, the central region of the partition 140 is provided with a support portion 145 in which a central groove 140a communicating with the procedure space 120a is formed. In this case, the support portion 145 protrudes from the first side surface 141 and has a cylindrical shape.

Meanwhile, a temperature sensor 250 for measuring the temperature of the procedure space 120a is provided above the central groove 140a. As such, since the temperature sensor 250 is positioned at the center of the procedure space 120a, it is arranged in a direction perpendicular to the raised skin of a user and can accurately measure the temperature around the raised skin.

In addition, the sensor unit 200 is disposed on the first side surface 141 of the partition 140. In addition, the sensor unit 200 detects changes in capacitance to sense a state in which a user's skin is in contact with the first electrode 131 and the second electrode 132 of the stimulation application unit 130. To this end, the sensor unit 200 includes a first sensor unit 210 having a ring shape and a second sensor unit 230 that is spaced apart from the first sensor unit 210.

The first sensor unit 210 is formed to surround the outer surface of the support portion 145 while being disposed on the first side surface 141. Accordingly, since the first sensor unit 210 maintains a non-contact state with a user's skin, there is no need to apply an electrical signal to the user's skin to detect whether the skin is raised, thereby preventing unnecessary electrical stimulation from being applied to the user's skin.

In addition, since the first sensor unit 210 is formed to surround the outer surface of the support portion 145, it does not interfere with the temperature measurement by the temperature sensor 250 and is also disposed adjacent to the raised skin of a user, thereby enabling precise measurement of the height of the raised skin.

In addition, since the first sensor unit 210 is formed to surround the outer surface of the support portion 145 and is disposed on the first side surface 141, it is positioned at a location corresponding to the raised skin of a user, thereby enabling precise measurement of the height of the raised skin.

The second sensor unit 230 is disposed above the support portion 145 so as to be spaced apart from the first sensor unit 210. In addition, the second sensor unit 230 receives power and detects capacitance using an electric field formed with the first sensor unit 210. Accordingly, the second sensor unit 230 measures changes in capacitance caused by the raised skin of a user based on the capacitance between the first sensor unit 210 and the second sensor unit 230.

Through this, the sensor unit 200 measures changes in capacitance caused by the raised skin and senses whether the raised skin is in contact with the first electrode 131 and the second electrode 132 of the stimulation application unit 130.

Accordingly, according to an embodiment of the present invention, if the contact area between a user's skin and the first electrode 131 and the second electrode 132 of the stimulation application unit 130 is too small, excessive application of high-frequency stimulation from the first electrode 131 and the second electrode 132 to the user's skin is prevented.

In addition, the support portion 145 is provided with a first support portion 221 that supports the temperature sensor 250. In this case, the support portion 145 is further provided with a second support portion 222 that is formed to surround the first support portion 221 and the temperature sensor 250. In this case, the second support portion 222 prevents air from flowing in or out between the through hole 140a and the interior of the main body 110.

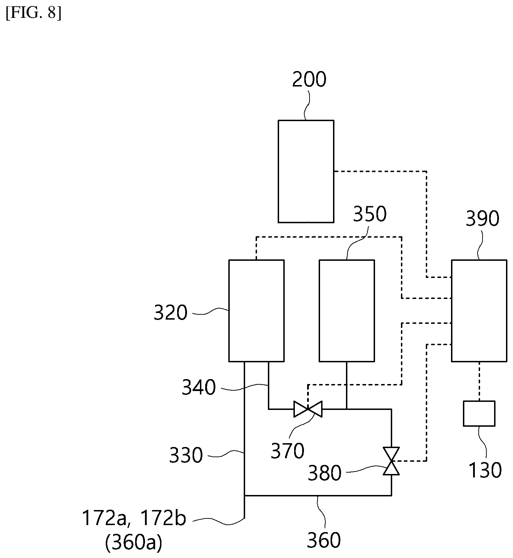

FIG. 8 is a block diagram of the cosmetic procedure device according to an embodiment of the present invention.

Referring to FIG. 8, the cosmetic procedure device according to an embodiment of the present invention may include a suction source 320, a pump discharge flow path 340, a chamber 350, a chamber discharge flow path 360, a first valve 370, a second valve 380, and a control unit 390.

The suction source 320 is disposed in the main body 110 and generates negative pressure. The suction source 320 may be disposed in an internal space of the main body 110. A suction function may be provided in which a user's skin is drawn toward the procedure space 120a (see FIG. 7) during a cosmetic procedure by the negative pressure generated by the suction source 320. For example, the suction source 320 may be a pneumatic pump.

The suction flow path 330 is connected to the suction source 320 such that external air is drawn into the suction source 320 by the negative pressure. In this case, the suction flow path 330 is provided with a plurality of suction ports 172a and 172b (see FIG. 2) that are arranged toward the procedure space 120a.

The plurality of suction ports 172a and 172b include a first suction port 172a formed on the second side surface 142, which is a surface opposite to the first side surface 141 of the partition 140 (see FIG. 2), and a second suction port 172b formed on the opposite side of the first suction port 172a with the support portion 145 therebetween.

Accordingly, when the suction source 320 operates during a cosmetic procedure, air present between the main body 110 and a user's skin is drawn into the suction flow path 330, causing the user's skin to be suctioned into the procedure space 120a.

The pump discharge flow path 340 is connected to the suction source 320 such that air drawn into the suction source 320 is discharged to the outside of the suction source 320. The pump discharge flow path 340 may be disposed inside the main body 110. When the suction source 320 operates, negative pressure is generated in the suction flow path 330, and air flows into the suction flow path 330 through the plurality of suction ports 172a and 172b. The air introduced into the suction flow path 330 flows into the suction source 320 and may be discharged to the outside of the suction source 320 through the pump discharge flow path 340.

The chamber 350 is connected to the pump discharge flow path 340, disposed in the main body 110, and stores air discharged through the pump discharge flow path 340. The chamber 350 may be disposed in an internal space of the main body 110. For example, the chamber 350 may be disposed adjacent to the suction source 320. While the suction source 320 is operating, air is drawn into the suction source 320 through the suction flow path 330, discharged to the outside of the suction source 320 through the pump discharge flow path 340, and then flows into the chamber 350, where it may be stored in the chamber 350.

The chamber discharge flow path 360 is connected to the chamber 350 so that air stored in the chamber 350 is discharged to the outside. Air stored in the chamber 350 may be discharged to the outside through the chamber discharge flow path 360. In this case, a discharge port 360a of the chamber discharge flow path 360 may be arranged to discharge air toward a user's skin.

In one embodiment of the present invention, the pump discharge flow path 360 may partially overlap with the suction flow path 330. For example, the pump discharge flow path 360 may be connected to one side of the suction flow path 330, and a portion of the pump discharge flow path 360 may overlap with a portion of the suction flow path 330. In this case, the discharge port 360a of the pump discharge flow path 360 may be the same as the plurality of suction ports 172a and 172b of the suction flow path 330. In other words, the discharge port 360a of the pump discharge flow path 360 may serve as the plurality of suction ports 172a and 172b of the suction flow path 330, and the plurality of suction ports 172a and 172b may serve as the discharge port 360a. This structure can maximize the efficiency of utilizing the internal space of the main body 110.

The first valve 370 is arranged to open and close the pump discharge flow path 340. For example, the first valve 370 may be a solenoid valve. The opening and closing of the first valve 370 may be performed by the control unit 390.

The first valve 370 is opened when the suction source 320 operates. Air drawn into the suction flow path 330 by the negative pressure generated by the suction source 320 flows through the suction source 320 and the pump discharge flow path 340 to the chamber 350. Meanwhile, when the first valve 370 is closed, the suction source 320 and the chamber 350 are blocked from each other.

The second valve 380 is arranged to open and close the chamber discharge flow path 360. For example, the second valve 380 may be a solenoid valve. The opening and closing of the second valve 380 may be performed by the control unit 390.

In one embodiment of the present invention, the second valve 380 may be opened while the first valve 370 is in a closed state. When the second valve 380 is opened, air stored in the chamber 350 flows into the pump discharge flow path 360 due to internal pressure and is discharged to the outside through the discharge port 360a of the pump discharge flow path 360. By discharging air through the discharge port 360a, a blowing function may be provided to a user during a cosmetic procedure. The air discharged through the discharge port 360a of the pump discharge flow path 360 may reach the user's skin and cool and soothe the skin.

Meanwhile, the second valve 380 may be closed while the suction source 320 is operating. As described above, when the suction source 320 operates, the first valve 370 may be opened, and air introduced into the suction flow path 330 by the operation of the suction source 320 flows through the suction source 320 and the pump discharge flow path 340 to the chamber 350. At this time, since the second valve 380 is closed, the air flowing into the chamber 350 is not discharged to the outside through the chamber discharge flow path 360 but is stored in the chamber 350.

The control unit 390 controls the suction source 320, the first valve 370, and the second valve 380. The control unit 390 may include one or more processors, memory elements, and the like capable of storing and processing data.

In addition, the control unit 390 is electrically connected to the sensor unit 200 and the stimulation application unit 130, and receives an electrical signal from the sensor unit 200 to control the operation of the stimulation application unit 130.

In addition, the control unit 390 is electrically connected to the temperature sensor 250 (see FIG. 2). And the temperature sensor 250 provides information that enables determination of whether discomfort due to heat or a risk of burns may occur, by measuring the temperature around a user's skin or the temperature of the user's skin during a cosmetic procedure.

In association with the temperature sensor 250, the control unit 390 may close the first valve 370 and open the second valve 380 to allow air stored in the chamber 350 to be discharged through the chamber discharge flow path 360, when the temperature measured by the temperature sensor 250 during a cosmetic procedure exceeds a preset reference temperature. In this case, the reference temperature may refer to a temperature at which a user may feel discomfort or may suffer a burn. When the temperature measured by the temperature sensor 250 is higher than the preset reference temperature, air inside the chamber 350 can be discharged toward the user's skin through this control operation, and the discharged air may cool and soothe the user's skin.

Although exemplary embodiments of the present invention have been described, the spirit of the present invention is not limited to the embodiments set forth herein. Those of ordinary skill in the art who understand the spirit of the present invention may easily propose other embodiments through supplement, change, removal, addition, etc. of elements within the same scope of spirit, but the embodiments will be also within the scope of the present invention.

## Claims

1. A cosmetic procedure device for performing a cosmetic procedure on a user's skin, comprising:
a main body;
a procedure unit provided in the main body, the procedure unit having a procedure space formed therein to perform a procedure on the user's skin;
a stimulation application unit disposed in the procedure space, the stimulation application unit applying stimulation to the user's skin; and
a sensor unit disposed in the procedure unit, the sensor unit sensing a state in which the user's skin is in contact with the stimulation application unit by detecting a change in capacitance.

2. The cosmetic procedure device of claim 1,
wherein the stimulation application unit comprises:
a first electrode; and
a second electrode disposed in parallel with and spaced apart from the first electrode.

3. The cosmetic procedure device of claim 2,
wherein the procedure unit further comprises a partition that separates an interior of the main body from the procedure space, and
the sensor unit is disposed on a first side surface of the partition.

4. The cosmetic procedure device of claim 3,
wherein a support portion having a central groove communicating with the procedure space is provided in a central region of the partition, and
a temperature sensor configured to measure the temperature of the procedure space is provided in the central groove.

5. The cosmetic procedure device of claim 4, wherein the sensor unit is formed to surround an outer surface of the support portion.

6. The cosmetic procedure device of claim 5,
wherein the sensor unit comprises:
a first sensor unit having a ring shape; and
a second sensor unit disposed to be spaced apart from the first sensor unit.

7. The cosmetic procedure device of claim 4,
further comprising:
a suction source disposed in the main body and configured to generate suction force;
a suction flow path that forms negative pressure in the procedure space by drawing in air from the procedure space using the suction force of the suction source; and
a control unit electrically connected to the sensor unit and configured to control the stimulation application unit and the suction source.

8. The cosmetic procedure device of claim 7,
wherein the suction flow path includes a plurality of suction ports arranged toward the procedure space, and
the plurality of suction ports comprise:
a first suction port formed on a second side surface of the partition, the second side surface being opposite to the first side surface of the partition; and
a second suction port formed on the opposite side of the first suction port with the support portion therebetween.
